# FASCICULE DE BREVET EUROPEEN

(11) **EP 0 668 997 B1**
(45) Date de publication et mention de la délivrance du brevet: **23.12.1998**
(21) Numéro de dépôt: 94925457.7
(22) Date de dépôt: 16.08.1994
(51) Int. Cl.: G01N 19/02, G01N 33/42, G01M 17/02

(54) **DISPOSITIF POUR LA MISE EN OEUVRE DE METHODES DE MESURES DE RESISTANCES AU ROULEMENT ET DE LIMITES D'ADHERENCE, ET LESDITES METHODES**
VERFAHREN UND VORRICHTUNG FÜR DIE VERWENDUNG VON MESSMETHODEN ZUR BESTIMMUNG DER ROLLREIBUNG UND DES HAFTUNGSGRENZWERTES
DEVICE FOR IMPLEMENTING METHODS FOR MEASURING ROLLING RESISTANCES AND GRIP LIMITS, AND METHODS IMPLEMENTED

(30) Priorité: 10.09.1993 FR 9310953
(43) Date de publication de la demande: 30.08.1995
(73) Titulaire: COMPAGNIE GENERALE DES ETABLISSEMENTS MICHELIN - MICHELIN & CIE, 63040 Clermont-Ferrand Cédex (FR)
(72) Inventeur: SIMON, Jean-René, F-63100 Clermont-Ferrand (FR)
(74) Mandataire: Devaux, Edmond-Yves
(86) Numéro de dépôt international: EP9402718
(87) Numéro de publication internationale: WO9507456

(56) Documents cités:
- FR-A- 1 227 807
- US-A- 3 367 170
- JOURNAL OF TESTING AND EVALUATION., vol.20, no.6, Novembre 1992, PHILADELPHIA US pages 470 - 474, XP000331199 SIEW-ANN TAN ET AL. 'LABORATORY WHEEL TRACKING APPARATUS FOR BITUMINOUS PAVEMENT STUDIES'
- SOVIET INVENTIONS ILLUSTRATED Week 8938, 6 Septembre 1989 Derwent Publications Ltd., London, GB; AN 89-218769/30 & SU,A,1 434 334 (SARAT POLY) 30 Octobre 1988

## Description

La présente invention concerne un dispositif permettant d'appréhender les efforts existant entre un pneumatique et le sol sur lequel il roule et permettant plus particulièrement une mesure des propriétés du pneumatique mettant en jeu les efforts longitudinaux. De telles propriétés sont par exemple la résistance au roulement et le coefficient d'adhérence longitudinal. Elle concerne aussi les méthodes mises en oeuvre à l'aide d'un tel dispositif et plus particulièrement les méthodes de mesures comparatives des propriétés de résistances au roulement et de limites d'adhérence de pneumatiques différents, ou des mêmes propriétés dues à des sols différents.

Compte tenu de tous les facteurs susceptibles d'intervenir au cours d'une mesure de résistance au roulement, celle-ci est très délicate et, comme connu, il existe deux grandes catégories de méthodes : la première catégorie concerne les mesures sur machines, et il faut citer les mesures sur volant, le pneumatique à tester roulant sur un volant de plus ou moins grand diamètre, et les mesures sur bancs à rouleaux, lesdits rouleaux ayant de petits diamètres et provoquant des sollicitations du pneumatique très éloignées de celles rencontrées sur des sols plans d'où l'apparition de machine sur sol plan, sous forme de bande sur laquelle roule le pneumatique ; la deuxième catégorie concerne les mesures sur pistes et sols réels qui se pratiquent généralement avec un véhicule que ce soit un véhicule analytique ou un véhicule classique. Avec ce dernier type de véhicule est souvent employée la méthode dite de décélération sur route, méthode sans doute moins précise que les autres mais donnant des résultats intéressants car intégrant au maximum les facteurs d'environnement.

Quant aux mesures de limites d'adhérence, elles se font aussi généralement à l'aide de volants lorsqu'il s'agit de mesures sur machines, mais plus fréquemment elles se réalisent à l'aide de véhicules roulant sur des pistes avec des sols variés et variables. Il est évident que les méthodes avec véhicules et pistes demandent des espaces considérables, de même que les méthodes avec volants car ceux-ci doivent avoir des grands diamètres.

L'invention a pour but de proposer un dispositif à la fois simple et permettant une mesure au moins comparative de résistances au roulement et/ou de limites d'adhérence dans un espace restreint, ou pour le moins très réduit par rapport à des pistes ou à des ateliers, tout en permettant un roulement sur sol plan ou de courbure très faible.

Le dispositif, conforme à l'invention, est caractérisé en ce qu'il comprend deux ensembles montés, dimensionnellement identiques, assemblés par un bras de liaison dont l'axe moyen est parallèle à la droite joignant les centres de rotation des ensembles montés non chargés, ledit bras de liaison étant pourvu de moyens de lestage nécessaires à l'obtention de la charge imposée à chacun des ensembles et tels que le centre de gravité du dispositif au repos soit localisé entre ladite droite et le sol sur lequel roulera le dispositif, celui-ci comprenant en outre des moyens susceptibles, par rotation des ensembles montés, de conférer à son centre de gravité un mouvement cycloïdal lui donnant une position angulaire déterminée par rapport au plan contenant la droite de jonction des centres de rotation des ensembles montés et perpendiculaire au sol.

Il faut entendre par ensemble monté l'ensemble formé d'au moins un pneumatique gonflé à une pression donnée, de sa jante de montage, ladite jante étant assortie d'un disque de roue, pouvant être fixé sur un moyeu. Le bras de liaison réunissant les deux ensembles montés peut être une barre de liaison rigide, de section transversale quelconque, par exemple circulaire ou carrée, et fixée solidement aux deux moyeux sur lesquels sont eux-mêmes fixés les disques. Ce bras sera avantageusement un bras pouvant tourner librement dans les logements prévus à cet effet dans les moyeux et disques des ensembles montés. Etant précisé qu'il faut comprendre que, dans le terme parallèle, est inclus le terme confondu, le bras de liaison sera préférentiellement l'essieu de rotation, dont l'axe moyen, qui est axe de symétrie, est la droite joignant les centres de rotation des ensembles montés.

Quant aux moyens susceptibles de conférer au centre de gravité du dispositif, radialement au dessous de la droite joignant les centres de rotation des ensembles montés, un mouvement cycloïdal, ils peuvent être de différentes sortes. Le mouvement de rotation des ensembles montés peut être le résultat de l'application d'une force de traction à l'essieu réunissant les centres de rotation des ensembles montés et tournant librement dans les moyeux. Ce mouvement peut être aussi le résultat de l'application aux deux ensembles d'un couple de rotation imposé manuellement ou mécaniquement. Il peut être aussi le résultat d'une inclinaison du sol sur lequel va rouler le dispositif.

De manière avantageuse, et dans le but d'obtenir une mesure comparative de résistances au roulement, le dispositif conforme à l'invention sera muni d'un bras de manutention qui fait fonction de levier, solidement fixé au(x) moyeu(x) ou au(x) disque(s) d'un (des) ensemble(s) monté(s), et de moyens susceptibles de maintenir le centre de gravité à la position angulaire désirée.

Préférentiellement, ce bras de manutention est, lorsque le dispositif est au repos, situé dans le plan contenant l'axe moyen de l'essieu liaison des centres de rotation des ensembles et perpendiculaire au sol sur lequel va rouler le dispositif, et fixé solidement à l'essieu réunissant les deux ensembles montés, l'essieu étant alors rotationnellement solidaire des moyeux, et les moyens de lestage étant solidaires de l'essieu.

Dans le cas de la mesure de limite d'adhérence d'un pneumatique donné, le dispositif, conforme à l'invention comprend un essieu réunissant les centres de rotation des ensembles, muni d'au moins une poulie d'enroulement d'un câble de traction, poulie fixée solidement et solidaire en rotation dudit essieu. L'application d'une force de traction au câble, par des moyens usuels et connus, permet la mise en rotation du dispositif, ainsi que la mise en mouvement du centre de gravité G du dispositif.

Conformément à l'invention, un premier procédé permettant la mesure comparative de résistances au roulement de deux trains de pneumatiques sur un même sol, consiste à utiliser deux dispositifs tels que décrits ci-dessus, un premier dispositif équipé d'un premier train de pneumatiques identiques entre eux, un deuxième dispositif équipé d'un deuxième train de pneumatiques identiques entre eux mais différents des pneumatiques équipant le premier dispositif, à placer les deux dispositifs sur le même sol, à assurer la charge nécessaire à chacun des dispositifs avec les moyens de lestage, à conférer aux centres de gravité des deux dispositifs un mouvement cycloïdal par la mise en rotation des deux dispositifs, jusqu'à obtention d'une même position angulaire des centres de gravité, à bloquer lesdits centres dans cette position et à leur donner au même instant la liberté de mouvement.

Lors du soulèvement identique des centres de gravité des deux dispositifs, dû au roulement desdits dispositifs, les mêmes énergies potentielles ont été emmagasinées. Lors de la libération des dispositifs, ceux-ci sont animés d'un mouvement pendulaire amorti et roulant, l'amortissement étant dû à la résistance au roulement des pneumatiques équipant les dispositifs, cet amortissement étant proportionnel à la résistance au roulement. Les dispositifs préférentiellement utilisés étant pourvus d'un bras de manutention provoquant la mise en rotation, le blocage des dispositifs dans une position donnée est avantageusement assuré par lesdits bras qui sont crochetés ou amarrés au sol.

La mesure comparative se fait préférentiellement par la comparaison du nombre d'oscillations subi respectivement par chaque dispositif, équipé d'un train de pneumatiques à comparer, nombre d'oscillations pour un intervalle de temps donné, ou nombre d'oscillations jusqu'à arrêt du dispositif.

Conformément à l'invention, un deuxième procédé permettant la mesure comparative des limites d'adhérence de trains de pneumatique sur un même sol consiste à utiliser au moins deux dispositifs tels que décrits, un premier dispositif étant équipé d'un premier train de pneumatiques identiques entre eux, le deuxième dispositif étant équipé d'un deuxième train de pneumatique identiques entre eux mais différents des pneumatiques du premier train, à placer les dispositifs sur le même sol, à assurer la charge nécessaire à chacun des dispositifs avec les moyens de lestage, à exercer une force de traction résultante sur chacun des dispositifs au moyen d'au moins un câble de traction enroulé sur une poulie d'enroulement solidairement fixée à l'essieu de rotation du dispositif, jusqu'à obtention du glissement entre le train de pneumatiques et le sol.

La comparaison des limites d'adhérence peut se faire par la vision des barres de manutention des deux dispositifs, qui servent alors d'aiguilles de mesure, mais préférentiellement elle se fera par lecture des forces appliquées, au moyen de dynamomètres placés sur le(s) câble(s) de traction.

Si l'on appelle les mesures comparatives décrites ci-dessus des mesures réalisées en parallèle, ces mêmes mesures peuvent aussi être réalisées en série à l'aide d'un seul dispositif, avec lequel on effectue une première mesure avec un premier train de pneumatiques puis une deuxième mesure avec un deuxième train de pneumatiques.

Les caractéristiques et avantages de l'invention seront mieux comprises à l'aide de la description qui suit et qui se réfère au dessin illustrant des exemples d'exécution et sur lequel,
- la figure 1 représente schématiquement une première variante du dispositif conforme à l'invention ;
- la figure 2 représente tout aussi schématiquement une deuxième variante dudit dispositif,
- la figure 3 représente schématiquement une troisième variante dudit dispositif ;
- la figure 4 représente schématiquement l'appareil montré sur la figure 1 et utilisé dans le procédé de mesure de résistances au roulement ;
- la figure 5 montre schématiquement l'appareil de la figure 3 et son utilisation dans le procédé de mesure de limites d'adhérence ;
- la figure 6 représente schématiquement l'appareil montré sur la figure 1 utilisé de manière différente dans le procédé de mesure de limites d'adhérence.

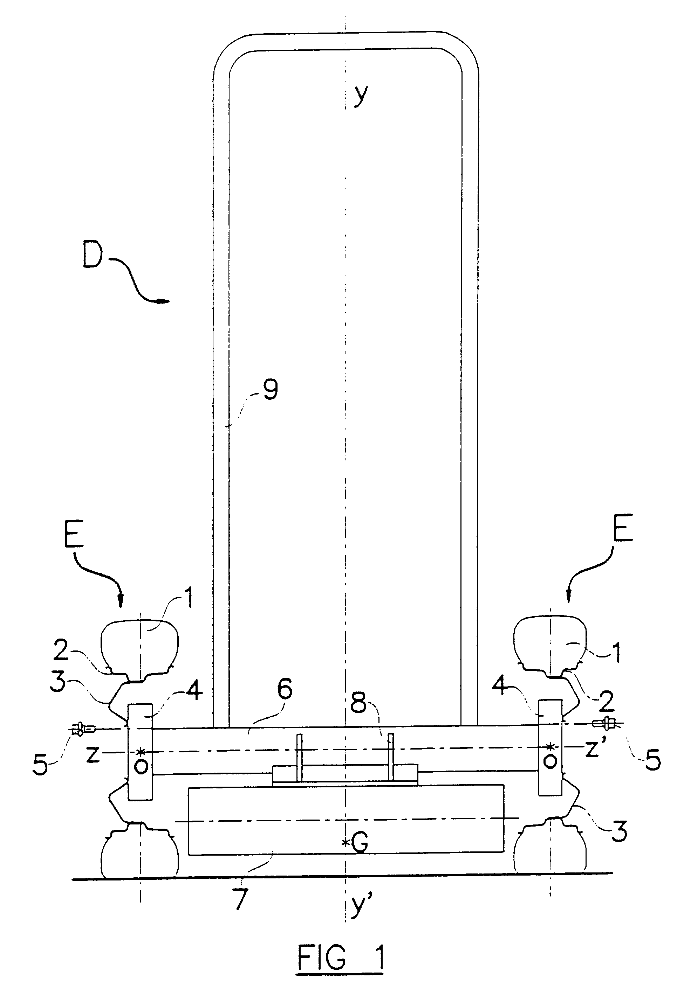

Le dispositif D, montré sur la figure 1 et conforme à l'invention, comprend deux ensembles montés identiques entre eux. Chacun de ces ensembles E est composé d'un pneumatique (1), monté sur sa jante de montage (2), et gonflé à sa pression d'essai. La jante (2) est réunie à un moyeu (4) par l'intermédiaire d'un disque (3), qui est assemblé au moyeu à l'aide de vis de fixation (5). Les deux moyeux (4) sont joints par un essieu (6) de section circulaire, qui, dans l'exemple décrit, est fixé solidement aux moyeux (4), l'essieu (6) étant dans l'impossibilité de tourner librement par rapport aux moyeux (4). L'essieu (6) est muni d'un porte-charge (7), qui est une nacelle ou un caisson dans lequel sont fixés des éléments de fonte ou gueuses destinés à assurer symétriquement la charge désirée sur les ensembles montés E. Ce caisson (7) est fixé solidement à l'essieu par le moyen de goussets (8), de sorte qu'à l'état de repos, le centre de gravité G du dispositif soit nettement radialement au dessous de l'axe ZZ' réunissant les deux centres de rotation O respectivement des ensembles montés E. L'essieu (6) est aussi muni d'un bras de manutention (9) qui est en fait une barre de forme de U renversé, cette barre étant solidement liée à l'essieu (6) et destinée à mettre en mouvement de rotation le dispositif D.

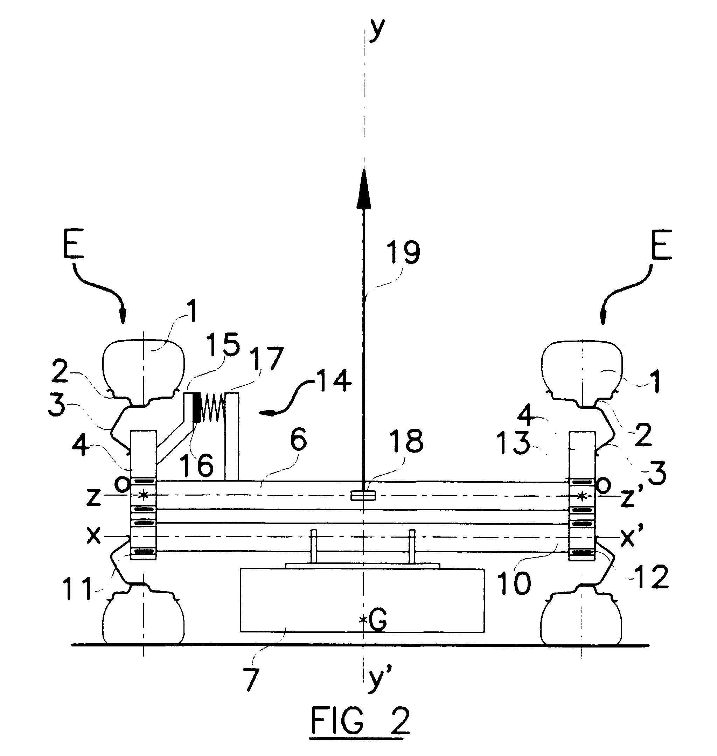

Une variante du dispositif conforme à l'invention est montrée sur la figure 2. Ce dispositif D diffère principalement de celui montré sur le figure 1 par le fait que le bras de liaison (10) sur lequel est fixé solidement le caisson porte-charge (7) et qui réunit les deux moyeux (4), (les mêmes références seront employées dans le cas d'éléments communs aux deux figures) n'a pas son axe de symétrie XX' confondu avec l'axe de symétrie ZZ' de l'essieu (6) joignant les deux centres de rotation O des ensembles montés E. Ce bras de liaison ou bras d'articulation peut tourner librement dans les logements (11) prévus à cet effet dans les moyeux (4), logements dans lesquels sont disposés les roulements (12). Il en est de même de l'essieu (6) qui, comme cela pourrait être le cas de la figure 1, peut tourner librement dans les logements (13), mais peut aussi être bloqué par un dispositif (14), composé de garnitures de friction (15) sur lesquelles viennent s'appliquer des plaquettes (16) sous l'effet de forces de serrage provoquées par la compression des ressorts (17). Le mouvement en rotation du dispositif selon la figure 2 est assuré par une force de traction s'exerçant sur un élément de traction (18) qui peut être une barre, une corde ou un filin métallique, fixé solidement à l'essieu (6), mais qui peut s'en détacher aisément et instantanément lorsque l'on désire libérer le dispositif. La force de traction est exercée par tout dispositif mécanique connu, tel qu'un treuil manuel ou mécanisé, ou plus simplement manuellement. Sur l'essieu (6), qui sera bloqué pendant la mesure de résistances au roulement, est fixée une aiguille de mesure (19) permettant de visualiser le mouvement perpendiculaire du centre de gravité G du dispositif, et de compter le nombre d'oscillations jusqu'à arrêt complet du mouvement pendulaire.

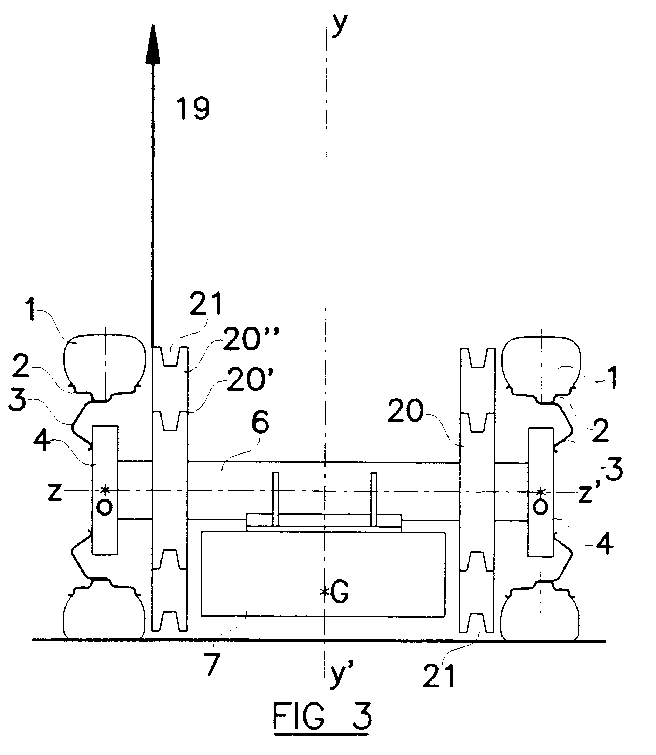

La variante du dispositif conforme à l'invention et montrée sur la figure 3 est sensiblement équivalente à celle montrée sur la figure 1. Elle en diffère cependant par les caractéristiques suivantes : le bras de liaison (10) qui est confondu avec l'essieu (6) de rotation du dispositif D est muni de deux poulies (20), pourvues elles-mêmes de gorges (21) dans lesquelles est enroulé un câble de traction. Ces poulies (20) sont bloquées sur l'essieu (6) par tout système d'attache approprié tel que vis, boulons...

Les poulies (20) peuvent être de diamètre variable, étant donné qu'elles sont avantageusement formées par la mise et la fixation sur une partie cylindrique (20') d'anneaux cylindriques (20") pourvus de gorges. Une des poulies (20) est munie d'une aiguille de mesure (19) comme décrit précédemment.

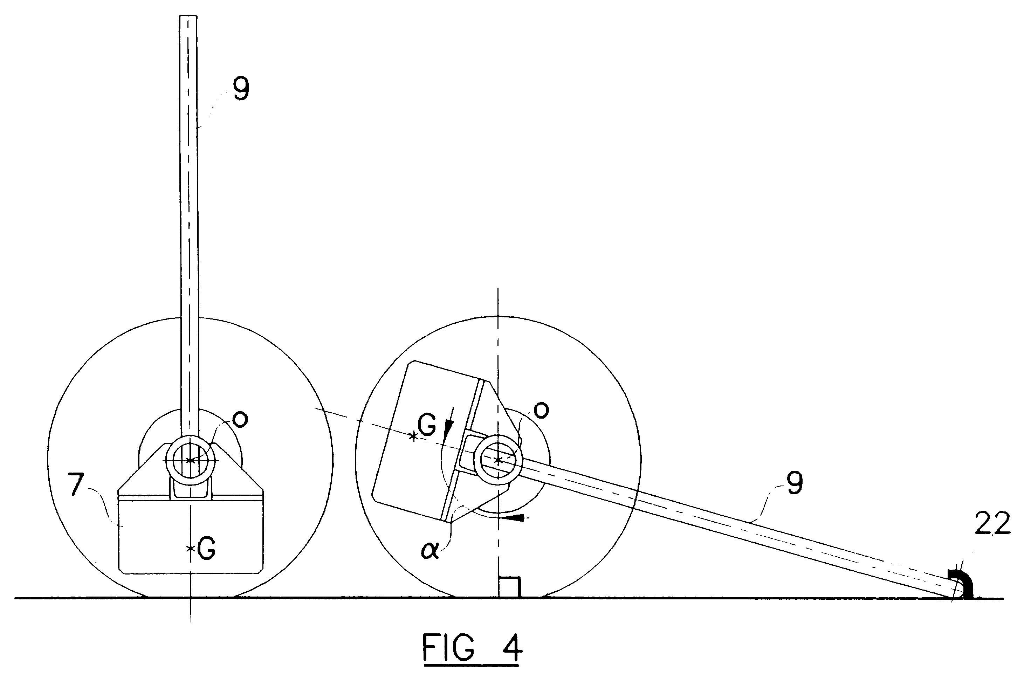

Sur la figure 4, le dispositif de la figure 1 est montré d'une part libre au repos et d'autre part après mise en position de départ du centre de gravité G du dispositif. Pour la mesure comparative des résistances au roulement de deux trains de pneumatiques, qui diffèrent entre eux soit par des caractéristiques de constitution, soit par des caractéristiques d'utilisation (pression, charge,...), deux dispositifs D, semblables à celui montré sur la figure 1, sont utilisés et placés l'un à côté de l'autre sur le même sol. Les deux dispositifs D sont animés d'un mouvement de rotation par application manuelle d'un couple de rotation au moyen de la barre de manutention (9) dont est équipé chaque dispositif, le mouvement de rotation étant poursuivi jusqu'à accrochage de la barre de manutention de chaque dispositif à un crochet (22) ancré au sol pendant cette rotation, les centres de gravité G (qui sont à la même distance des axes de rotation 00 si les charges sont égales pour les deux dispositifs) ou G' et G" (qui seront à des distances différentes de axes 00 si les charges sont inégales entre les deux dispositifs) décrivent des cycloïdes plus ou moins raccourcies, de telle façon que chaque centre de gravité définisse avec la trace 0, sur le plan de la figure, des axes 00, une droite faisant avec la direction perpendiculaire au sol et passant par le centre 0 un angle α identique pour les deux dispositifs, lorsque les barres (9) sont crochetées au moyen des crochets (22) fixés au sol. Au même instant T, les deux barres de manutention (9) sont décrochées, et les deux dispositifs sont alors animés d'un mouvement pendulaire, qui s'amortit en fonction des résistances au roulement globales, parmi lesquelles les résistances au roulement des pneumatiques.

Afin d'avoir une comparaison sûre, il est recommandé d'opérer avec des permutations de trains de pneumatiques : une première mesure s'effectue avec les pneumatiques A sur le dispositif D₁, alors que les pneumatiques B sont montés sur le dispositif D₂, et une deuxième mesure étant immédiatement réalisée après la première avec les pneumatiques A sur le dispositif D₂, et une deuxième mesure étant immédiatement réalisée après la première avec les pneumatiques A sur le dispositif D₂ et les pneumatiques B sur le dispositif D₁. La différence de résistances au roulement mesurée entre les deux trains peut s'apprécier par exemple par le nombre d'oscillations réalisé par les barres (9) respectivement des deux dispositifs jusqu'à arrêt complet des mouvements pendulaires, les distances totales parcourues des extrémités des barres (9) radialement les plus éloignées des centres de rotation 0 étant inversement proportionnelles aux résistances au roulement des pneumatiques A et B.

Il est bien évident que le procédé décrit pour comparer deux trains de pneumatiques est tout aussi valable pour comparer deux types ou plusieurs types de sol, différents par leurs caractéristiques, et en particulier les caractéristiques qui ont une influence sur la résistance au roulement. Il suffit de prendre quatre pneumatiques de référence, identiques entre eux, et qui sont préférentiellement des bandages caoutchouteux, pleins de faible hauteur, et de faire rouler simultanément les deux ou plusieurs dispositifs D sur les deux ou plusieurs sols différents.

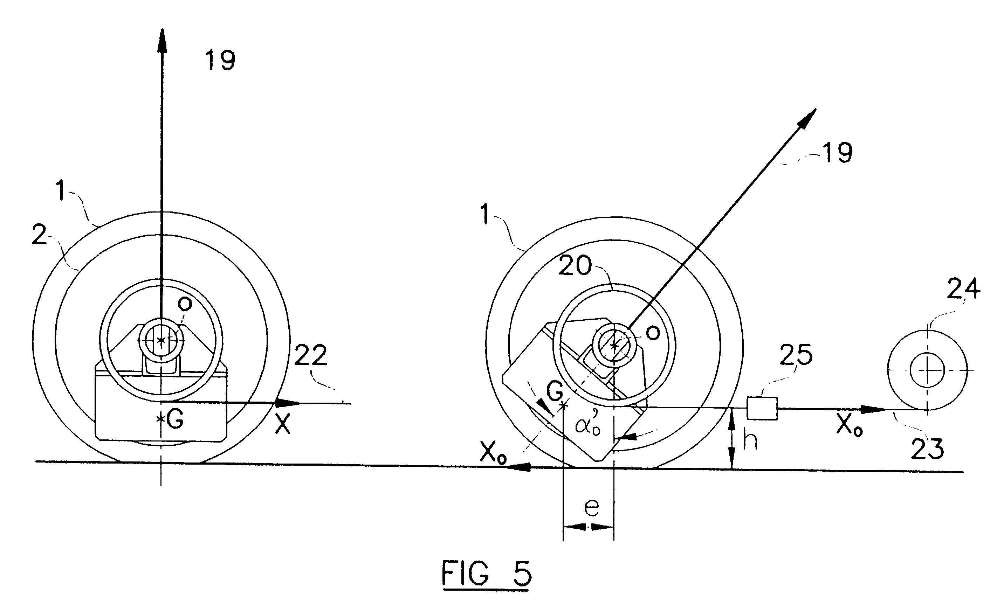

Sur la figure 5, sont rendues compréhensibles les conditions d'utilisation des deux ou plusieurs dispositifs nécessaires pour comparer les limites d'adhérence de deux ou plusieurs trains de pneumatiques. Les dispositifs utilisés sont préférentiellement les dispositifs montrés sur la figure 3. Le procédé de mesure diffère principalement du précédent par le fait qu'on applique à chaque dispositif un couple de rotation par l'intermédiaire d'une force de traction résultante X, appliquée sur des câbles de traction (23), enroulés dans les gorges de deux poulies (20) du dispositif de la figure 3, poulies qui sont disposées symétriquement par rapport au plan de symétrie YY'. Dans un premier temps, l'application de cette force X, au moyen d'un treuil (24), à une distance h du sol plan dans l'exemple décrit, provoque le mouvement de rotation du dispositif autour de l'axe de rotation 00 de sorte que le centre de gravité G du dispositif, vu en section dans le plan de symétrie YY' du dispositif, soit situé sur une droite GO faisant avec la perpendiculaire au sol passant par 0 un angle α', angle nul au départ du mouvement et qui croîtra en fonction de l'effort de traction appliqué X jusqu'à atteindre une valeur α'₀ pour lequel il y aura glissement des ensembles montés sur le sol considéré, la limite d'adhérence étant alors atteinte pour le train de pneumatiques considéré, et la force de traction étant alors X₀. L'angle α'₀ correspond à une certaine distance axiale e entre le centre de gravité G et la droite portant la réaction au sol égale à la charge portée, il est possible d'en déduire le coefficient d'adhérence à la limite d'adhérence, comme étant égale au rapport de la distance axiale e sur la distance radiale h.

La mesure comparative peut être aisément visionnée par les positions respectives des deux aiguilles (19) de mesure, mais peut aussi être avantageusement réalisée à l'aide de dynamomètres (25) placés sur les câbles de traction (22), donnant les valeurs des efforts X₀ respectivement pour les deux trains de pneumatiques.

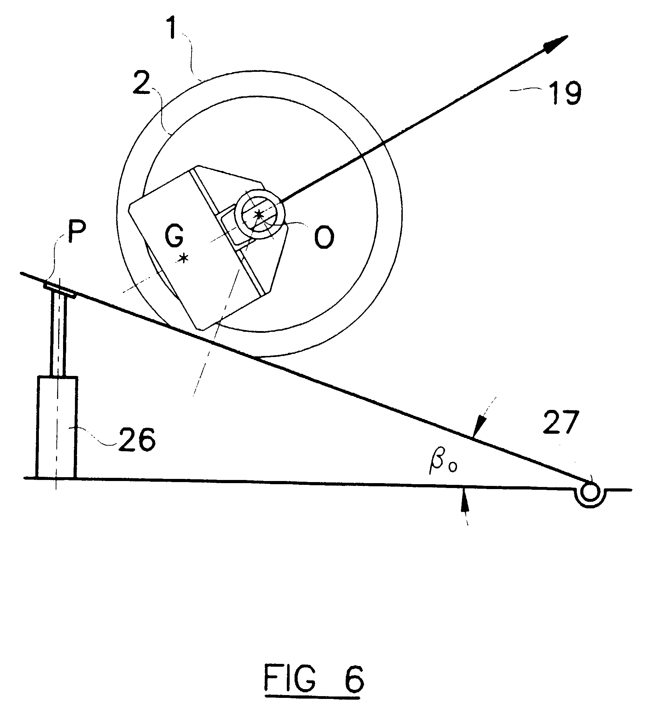

La figure 6 concerne aussi une mesure comparative des limites d'adhérence, avec utilisation de dispositifs, conformes à l'invention, et plus particulièrement de dispositifs tels que montrés sur les figures 1 ou 2. Le procédé diffère du procédé décrit précédemment en relation avec la figure 5 par le fait que le mouvement de rotation initial des dispositifs D et le glissement des ensembles montés E sur le sol étudié est provoqué, non par une force de traction X horizontale exercée sur un ou des câbles de traction (22) par un système approprié, mais par l'inclinaison β de plans inclinés de roulement P, ladite inclinaison étant variable par le fait que les plans inclinés P peuvent être animés d'un mouvement de rotation autour d'axes (27) situés au niveau du sol, par le moyen de vérins (26), par exemple.

Les dispositifs D sont placés initialement sur les plans de roulement P à l'état horizontal. Les plans P sont alors inclinés à vitesse constante et lente, chaque dispositif se met alors en rotation, puis pour un angle β₀ de son plan de roulement, le dispositif glisse sur le plan. La mesure comparative des deux limites d'adhérence peut être réalisée à l'aide des aiguilles de mesure (19), mais il est aussi possible de comparer par les angles respectifs β₀ des deux plans de roulement P. La connaissance de ces angles permet par ailleurs le calcul des forces de frottement respectives des deux trains de pneumatiques.

Dans le cadre des mesures de limites d'adhérence, il est aussi évident que les comparaisons peuvent porter, non plus sur des trains de pneumatiques, mais sur des sols différents par leurs caractéristiques que ce soit de sols plans ou des plans inclinés, et en particulier sur des sols différents par leur lubrification.

Il est tout aussi évident que la dispersion des mesures est minimisée par des permutations de trains de pneumatiques sur les dispositifs utilisés ainsi que la permutation de dispositifs sur les sols à comparer.

## Revendications

1. Dispositif D destiné à la mesure des résistances au roulement et/ou des limites d'adhérence de trains de pneumatiques (1) caractérisé en ce qu'il comprend au moins deux ensembles montés E identiques, assemblés par un bras de liaison (10) dont l'axe moyen XX' est parallèle à la droite ZZ' joignant les centres de rotation des ensembles montés non chargés, ledit bras de liaison (10) étant pourvu de moyens de lestage (7) nécessaires à l'obtention de la charge imposée à chacun des ensembles E et tels que le centre de gravité G du dispositif D soit localisé au repos entre ladite droite ZZ' et le sol sur lequel roulera le dispositif, celui-ci comprenant en outre des moyens (9, 18, 20, P) susceptibles par rotation des ensembles montés E de conférer à son centre de gravité G un mouvement cycloïdal lui permettant de prendre une position angulaire déterminée par rapport au plan contenant la droite XX' de jonction des centres de rotation 0 des ensembles montés et perpendiculaire au sol.

2. Dispositif selon la revendication 1, caractérisé en ce que le bras de liaison (10) est confondu avec l'essieu (6) d'axe ZZ' joignant les centres de rotation 0 des ensembles E.

3. Dispositif selon la revendication 2, caractérisé en ce que l'essieu (6) est muni de moyens lui permettant de tourner librement dans les moyeux (4) des ensembles, ainsi que des moyens (14, 15, 16, 17), lui permettant d'être solidaire en rotation des moyeux (4).

4. Dispositif selon l'une des revendications 1 à 3, caractérisé en ce que les moyens susceptibles de conférer à son centre de gravité G un mouvement cycloïdal sont des moyens (18) permettant l'application d'une force de traction à l'essieu (6) de rotation du dispositif D.

5. Dispositif selon l'une des revendications 1 à 3, caractérisé en ce que les moyens susceptibles de conférer à son centre de gravité G un mouvement cycloïdal sont des moyens (9, 20) permettant l'application d'un couple de rotation aux deux ensembles E.

6. Dispositif selon la revendication 4, caractérisé en ce que les moyens (18) permettant l'application d'une force de traction comprend un élément de traction (18), fixé sur l'axe moyen ZZ' de l'essieu (6) tournant librement dans les moyeux (4), tout en étant détachable dudit axe, et la force de traction étant assurée manuellement ou par un dispositif mécanique.

7. Dispositif selon la revendication 5, caractérisé en ce que le moyen (9) permettant l'application d'un couple de rotation est une barre de manutention (9) agissant comme bras de levier sur lequel est appliqué une force d'origine manuelle ou mécanique, et en ce qu'il comprend des moyens (22) susceptibles de maintenir ledit centre de gravité G à la position angulaire désirée.

8. Dispositif selon la revendication 5, caractérisé en ce que les moyens (20) permettant l'application du couple de rotation sont des éléments de traction (22) enroulés dans les gorges (21) de poulies (20), de diamètre non nul tel que les points d'application des forces de traction appliquées soient situés dans un plan contenant les axes XX' et ZZ'.

9. Dispositif selon l'une des revendications 1 à 3, caractérisé en ce que les moyens susceptibles de conférer à son centre de gravité G un mouvement cycloïdal est un plan de roulement P incliné par rapport au sol, l'angle d'inclinaison β étant variable en fonction du temps.

10. Procédé de mesure comparative des résistances au roulement d'au moins deux trains de pneumatiques sur un même sol, caractérisé en ce qu'on utilise au moins deux dispositifs selon l'une des revendications 1 à 7, un premier dispositif étant équipé d'un premier train de pneumatiques A identiques entre eux, un deuxième dispositif D₂ étant équipé d'un deuxième train de pneumatiques B identiques entre eux mais différents de ceux A équipant le premier dispositif D₁, et en ce qu'on place les deux dispositifs D sur le même sol, on dispose dans les caissons porte-charges (7) les charges nécessaires à chacun des dispositifs D, on confère aux centres de gravité G des deux dispositifs D un mouvement cycloïdal par la mise en rotation des deux dispositifs, jusqu'à obtention de la même position angulaire α des deux centres de gravité G par rapport respectivement à chaque plan perpendiculaire à la surface du sol et contenant l'axe de rotation 00 de chaque dispositif D, on bloque lesdits centres de gravité G dans ladite position α et on leur donne au même instant la liberté de mouvement, les amortissements des mouvements pendulaires respectivement des deux dispositifs étant comparés par simple vision ou par mesures.

11. Procédé de mesure comparative des propriétés d'au moins deux surfaces de roulement, caractérisé en ce qu'on utilise au moins deux dispositifs D selon l'une des revendications 1 à 9, lesdits dispositifs étant tous équipés de bandages caoutchouteux, pleins, de faible hauteur sur jante, identiques entre eux, en ce qu'on dispose chaque dispositif sur une des surfaces de roulement à comparer , on confère aux centres de gravité G desdits dispositifs préalablement chargés, un mouvement cycloïdal par la mise en rotation des deux dispositifs D jusqu'à obtention de la même position angulaire a des deux centres de gravité G par rapport respectivement à chaque plan perpendiculaire à la surface du sol et contenant l'axe de rotation 00 de chaque dispositif D, on bloque lesdits centres de gravité G dans ladite position et on leur donne au même instant la liberté de mouvement, les amortissements des mouvements pendulaires respectivement des deux dispositifs étant comparés par simple vision ou par mesures.

12. Procédé de mesure comparative des limites d'adhérence d'au moins deux trains de pneumatiques sur un même sol, caractérisé en ce qu'on utilise au moins deux dispositifs selon la revendication 8, un premier dispositif D₁ étant équipé d'une première paire de pneumatiques A identiques entre eux, un deuxième dispositif D₂ étant équipé d'une deuxième paire de pneumatiques B identiques entre eux mais différents des pneumatiques équipant le dispositif D₁, on place les deux dispositifs chargés sur un même sol, on applique à chaque dispositif un couple de rotation par l'intermédiaire d'une force de traction résultante X₀, exercée sur des câbles de traction (22) enroulés dans les gorges (21) de deux poulies (20) disposées symétriquement par rapport au plan de symétrie YY' du dispositif, à une distance h du sol et permettant le glissement sur le sol.

13. Procédé de mesure comparative des limites d'adhérence d'au moins deux trains de pneumatiques sur un même sol, caractérisé en ce qu'on utilise au moins deux dispositifs selon la revendication 9, un premier dispositif D₁ étant équipé d'une première paire de pneumatiques A identiques entre eux, un deuxième dispositif D₂ étant équipé d'une deuxième paire de pneumatiques B identiques entre eux mais différents de pneumatiques équipant le dispositif D₁, on place chaque dispositif sur une surface de roulement P plan et horizontale, on incline chaque surface P jusqu'à obtention du glissement du dispositif correspondant à ladite surface, les limites d'adhérence étant mesurées par les angles d'inclinaison des deux plans inclinés et la comparaison portant sur les angles mesurés.

## Patentansprüche

1. Vorrichtung D, die zur Messung von Rollwiderständen und/oder Haftungsgrenzen von Reifensätzen (1) bestimmt ist,
**dadurch gekennzeichnet, daß**
sie mindestens zwei identische, montierte Baugruppen E aufweist, die durch einen Verbindungsarm (10) zusammenmontiert sind, dessen Mittelachse XX' parallel zur Geraden ZZ' ist, die die Drehmitten der unbelasteten, montierten Baugruppen verbindet, wobei der genannte Verbindungsarm (10) mit Beschwerungsmitteln (7) versehen ist, die notwendig sind, um die Belastung zu erzielen, die auf jede der Baugruppen E aufgebracht wird, und die so beschaffen sind, daß der Schwerpunkt G der Vorrichtung D in der Ruhelage zwischen der genannten Geraden ZZ' und dem Boden angeordnet ist, auf dem die Vorrichtung abrollen soll, und daß diese außerdem Mittel (9, 18, 20, P) aufweist, die imstande sind, durch Drehung der montierten Baugruppen E auf den Schwerpunkt G eine zykloidenartige Bewegung aufzubringen, die es ihm gestattet, eine bestimmte Winkellage einzunehmen, die in Bezug auf die Ebene bestimmt ist, die die Gerade XX' der Verbindung der Drehungsmitten O der montierten Baugruppen sowie die Senkrechte zum Boden enthält.

2. Vorrichtung nach Anspruch 1, dadurch gekennzeichnet, daß der Verbindungsarm (10) mit der Achse (6) der geometrischen Achse ZZ' zusammenfällt, die die Drehungsmitten O der Baugruppen E verbindet.

3. Vorrichtung nach Anspruch 2, dadurch gekennzeichnet, daß die Achse (6) mit Mitteln versehen ist, die es ihr gestatten, sich in den Naben (4) der Baugruppen frei zu drehen, sowie mit Mitteln (14, 15, 16, 17), die es ihr gestatten, drehfest mit den Naben (4) verbunden zu sein.

4. Vorrichtung nach einem der Ansprüche 1 bis 3, dadurch gekennzeichnet, daß die Mittel, die imstande sind, ihrem Schwerpunkt G eine zykloidenförmige Bewegung mitzuteilen, Mittel (18) sind, die es gestatten, eine Schleppkraft auf die Achse (6) zur Drehung der Vorrichtung D aufzubringen.

5. Vorrichtung nach einem der Ansprüche 1 bis 3, dadurch gekennzeichnet, daß die Mittel, die imstande sind, auf ihren Schwerpunkt G eine zykloidenförmige Bewegung aufzubringen, Mittel (9, 20) sind, die es gestatten, ein Drehmoment auf die beiden Baugruppen E aufzubringen.

6. Vorrichtung nach Anspruch 4, dadurch gekennzeichnet, daß die Mittel (18), die die Anwendung einer Schleppkraft gestatten, ein Zugelement (18) umfassen, das auf der geometrischen Mittelachse ZZ' der Achse (6) befestigt ist, die sich frei in den Naben (4) dreht, von der genannten geometrischen Achse jedoch lösbar ist, und daß die Zugkraft von Hand oder durch eine mechanische Vorrichtung sichergestellt ist.

7. Vorrichtung nach Anspruch 5, dadurch gekennzeichnet, daß die Mittel (9), die die Aufbringung eines Drehmoments gestatten, eine Förderstange (9) sind, die wie ein Hebelarm wirksam ist, auf den eine Kraft aufgebracht wird, deren Ursprung von Hand oder mechanisch ist, und daß sie Mittel (22) aufweist, die befähigt sind, den genannten Schwerpunkt G in der gewünschten Winkellage zu halten.

8. Vorrichtung nach Anspruch 5, dadurch gekennzeichnet, daß die Mittel (20), die die Aufbringung eines Drehmoments gestatten, Zugelemente (22) sind, die in Nuten (21) von Seilscheiben (20) aufgewickelt sind, mit einem Durchmesser, der nicht Null ist, so daß die Punkte zur Anbringung von Zugkräften, die aufgebracht wurden, in einer Ebene liegen, die die Achsen XX' und ZZ' enthält.

9. Vorrichtung nach einem der Ansprüche 1 bis 3, dadurch gekennzeichnet, daß die Mittel, die befähigt sind, ihrem Schwerpunkt G eine zykloidenförmige Bewegung mitzuteilen, eine Abrollebene P sind, die in Bezug auf den Boden geneigt ist, wobei der Neigungswinkel β in Funktion der Zeit variabel ist.

10. Vergleichendes Meßverfahren für die Rollwiderstände mindestens zweier Reifensätze auf demselben Boden, dadurch gekennzeichnet, daß man mindestens zwei Vorrichtungen gemäß einem der Ansprüche 1 bis 7 verwendet, wobei eine erste Vorrichtung mit einem ersten Satz von Reifen A ausgestattet ist, die untereinander identisch sind, und die zweite Vorrichtung D₂ mit einem zweiten Satz von Reifen B ausgestattet ist, die untereinander identisch sind, aber verschieden von den Reifen A, die die Ausstattung der ersten Vorrichtung D₁ bilden, und daß man die beiden Vorrichtungen D auf denselben Boden aufsetzt, man in den Last-Tragekästen (7) Lasten anordnet, die für jede der Vorrichtungen D notwendig sind, man den Schwerpunkten G der beiden Vorrichtungen D dadurch eine zykloidenförmige Bewegung verleiht, daß man die beiden Vorrichtungen in Drehung versetzt, bis man dieselbe Winkelposition α der beiden Schwerpunkte G in Bezug auf jede entsprechende Ebene erreicht, die senkrecht zur Oberfläche des Bodens steht und die Drehachse 00 einer jeden Vorrichtung D enthält, man die genannten Schwerpunkte G in der genannten Lage α blockiert und man ihnen im gleichen Augenblick die Bewegungsfreiheit verleiht, wobei die Abschwächungen der senkrechten Bewegungen jeweils der beiden Vorrichtungen durch einfache Sicht oder durch Messungen verglichen werden.

11. Vergleichendes Meßverfahren für die Eigenschaften mindestens zweier Rolloberflächen, dadurch gekennzeichnet, daß man mindestens zwei Vorrichtungen D nach einem der Ansprüche 1 bis 9 verwendet, wobei die genannten Vorrichtungen alle mit untereinander identischen, massiven Gummimänteln mit geringer Höhe über der Felge versehen sind, daß man jede Vorrichtung auf einer der zu vergleichenden Rolloberflächen anordnet, man den Schwerpunkten G der genannten Vorrichtungen, die vorher mit einer Last versehen wurden, dadurch eine zykloidenförmige Bewegung verleiht, daß man die beiden Vorrichtungen D in Drehung versetzt, bis man die gleiche Winkellage α der beiden Schwerpunkte G in Bezug auf jede jeweilige Ebene senkrecht zur Oberfläche des Bodens erhält, die die Drehachse 00 jeder Vorrichtung D enthält, man die genannten Schwerpunkte G in der genannten Lage blockiert und man ihnen im gleichen Augenblick die Bewegungsfreiheit verleiht, wobei die Abschwächungen der senkrechten Bewegungen jeweils der beiden Vorrichtungen durch einfache Sicht oder durch Messung verglichen werden.

12. Vergleichendes Meßverfahren für die Haftungsgrenzen mindestens zweier Sätze von Reifen auf demselben Boden, dadurch gekennzeichnet, daß man mindestens zwei Vorrichtungen nach dem Anspruch 8 verwendet, wobei eine erste Vorrichtung D₁ mit einem ersten Paar von Reifen A ausgestattet ist, die untereinander identisch sind, eine zweite Vorrichtung D₂ mit einem zweiten Paar von Reifen B ausgestattet ist, die untereinander identisch sind, sich aber von den Reifen unterscheiden, die die Vorrichtung D₁ ausstatten, man die beiden belasteten Vorrichtungen auf demselben Boden anordnet, man auf jede Vorrichtung ein Drehmoment mittels einer resultierenden Zugkraft X₀ aufbringt, die auf Zugseilen (22) ausgeübt wird, die in den Nuten (21) zweier Seilscheiben (20) aufgewickelt sind, die in Bezug auf die Symmetrieebene YY' der Vorrichtung symmetrisch angeordnet sind, unter einem Abstand h vom Boden, und man das Rutschen auf dem Boden gestattet.

13. Vergleichende Meßvorrichtung für die Haftungsgrenzen mindestens zweier Sätze von Reifen auf demselben Boden, dadurch gekennzeichnet, daß man mindestens zwei Vorrichtungen nach Anspruch 9 verwendet, wobei eine erste Vorrichtung D₁ mit einem ersten Paar von Reifen A ausgestattet ist, die identisch sind untereinander, und eine zweite Vorrichtung D₂ mit einem zweiten Paar von Reifen B ausgestattet ist, die untereinander identisch sind, sich aber von den Reifen unterscheiden, die die Vorrichtung D₁ ausstatten, man jede Vorrichtung auf einer Rollfläche P aufsetzt, die eben und horizontal ist, und man jede Oberfläche P neigt, bis man ein Rutschen der Vorrichtung entsprechend der genannten Oberfläche erzielt, wobei die Haftungsgrenzen durch die Neigungswinkel der beiden geneigten Ebenen gemessen werden und der Vergleich auf den gemessenen Winkeln beruht.

## Claims

1. A device D intended for measuring the rolling resistances and/or the limits of adhesion of sets of tyres (1), characterised in that it comprises at least two identical mounted assemblies E joined by a connecting arm (10), the central axis XX' of which is parallel to the straight line ZZ' joining the centres of rotation of the mounted, unloaded assemblies, said connecting arm (10) being provided with ballasting means (7) necessary for obtaining the load applied to each of the assemblies E and such that the centre of gravity G of the device D is located at rest between said straight line ZZ' and the ground on which the device will roll, the device also comprising means (9, 18, 20, P) capable by rotating the mounted assemblies E of imparting a cycloidal movement to its centre of gravity G which permits it to take up a definite angular position in relation to the plane containing the straight line XX' which joins the centres of rotation O of the mounted assemblies and which is perpendicular to the ground.

2. A device according to claim 1, characterised in that the connecting arm (10) is identical to the axle (6) with axis ZZ' joining the centres of rotation O of the assemblies E.

3. A device according to claim 2, characterised in that the axle (6) is provided with means permitting it to turn freely in the hubs (4) of the assemblies, and with means (14, 15, 16, 17) permitting it to be interlocked in rotation with the hubs (4).

4. A device according to any one of claims 1 to 3, characterised in that the means capable of imparting a cycloidal movement to its centre of gravity G are means (18) permitting the application of a pulling force to the axle (6) of rotation of the device D.

5. A device according to any one of claims 1 to 3, characterised in that the means capable of imparting a cycloidal movement to its centre of gravity G are means (9, 20) permitting the application of a turning moment to the two assemblies E.

6. A device according to claim 4, characterised in that the means (18) permitting the application of a pulling force comprises a traction element (18) fixed to the central axis ZZ' of the axle (6) turning freely in the hubs (4) while being detachable from said axis, the pulling force being provided manually or by a mechanical device.

7. A device according to claim 5, characterised in that the means (9) permitting the application of a turning moment is a handling bar (9) acting as a lever arm to which a force of manual or mechanical origin is applied, and in that it comprises means (22) capable of maintaining said centre of gravity G at the desired angular position.

8. A device according to claim 5, characterised in that the means (20) permitting the application of the turning moment are traction elements (22) wound in the grooves (21) of pulleys (20), of a diameter other than zero such that the points of application of the pulling forces applied are situated in a plane containing the axes XX' and ZZ'.

9. A device according to any one of claims 1 to 3, characterised in that the means capable of imparting a cycloidal movement to its centre of gravity G is a rolling plane P inclined in relation to the ground, the angle of inclination β being variable as a function of time.

10. A comparative method of measuring the rolling resistances of at least two sets of tyres on the same ground, characterised in that at least two devices according to any one of claims 1 to 7 are used, a first device being fitted with a first set of tyres A which are identical to each other, a second device D₂ being fitted with a second set of tyres B which are identical to each other but different from those A with which the first device D₁ is fitted, and in that the two devices D are placed on the same ground, the loads necessary for each of the devices D are disposed in the load-carrying vessels (7), a cycloidal movement is imparted to the centres of gravity G of the two devices D by setting the two devices in rotation until the same angular position α of the two centres of gravity G is obtained in relation to each respective plane perpendicular to the surface of the ground and containing the axis of rotation 00 of each device D, said centres of gravity G are locked in said position α and they are given freedom of movement at the same moment, the damping of the respective oscillating movements of the two devices being compared simply visually or by measurement.

11. A comparative method of measuring the properties of at least two rolling surfaces, characterised in that at least two devices D according to any one of claims 1 to 9 are used, said devices all being fitted with rubber tyres, which are solid, of low height above the rim, and which are identical to each other, in that each device is disposed on one of the rolling surfaces to be compared, a cycloidal movement is imparted to the centres of gravity G of said pre-loaded devices by setting the two devices D in rotation until the same angular position α of the two centres of gravity G is obtained in relation to each respective plane perpendicular to the surface of the ground and containing the axis of rotation 00 of each device D, said centres of gravity G are locked in said position and they are given freedom of movement at the same moment, the damping of the respective oscillating movements of the two devices being compared simply visually or by measurement.

12. A comparative method of measuring the limits of adhesion of at least two sets of tyres on the same ground, characterised in that at least two devices according to claim 8 are used, a first device D₁ being fitted with a first pair of tyres A which are identical to each other, a second device D₂ being fitted with a second pair of tyres B which are identical to each other but different from the tyres with which the first device D₁ is fitted, the two loaded devices are placed on the same ground, a turning moment is applied to each device by means of a resultant pulling force X₀ exerted on traction cables (22) wound in the grooves (21) of two pulleys (20) symmetrically disposed in relation to the plane of symmetry YY' of the device, at a distance h from the ground and permitting sliding on the ground.

13. A comparative method of measuring the limits of adhesion of at least two sets of tyres on the same ground, characterised in that at least two devices according to claim 9 are used, a first device D₁ being fitted with a first pair of tyres A which are identical to each other, a second device D₂ being fitted with a second pair of tyres B which are identical to each other but different from the tyres with which the first device D₁ is fitted, each device is placed on a plane, horizontal rolling surface P, each surface P is inclined until sliding of the device corresponding to said surface is obtained, the limits of adhesion being measured by the angles of inclination of the two inclined planes and the comparison being based on the angles measured.
